# EUROPÄISCHE PATENTANMELDUNG

(11) **EP 2 904 997 A1**
(43) Veröffentlichungstag der Anmeldung: **12.08.2015**
(21) Anmeldenummer: 14000428.4
(22) Anmeldetag: 05.02.2014
(51) Int. Cl.: A61F 5/56

(54) **Okklusionsschiene**

(71) Anmelder: Bruderhofer, Andreas, 81379 München (DE); Kursawe, Leon, 81379 München (DE)
(72) Erfinder: Bruderhofer, Andreas, 81379 München (DE); Kursawe, Leon, 81379 München (DE)
(74) Vertreter: Heuer, Wilhelm

(57) **Zusammenfassung**

Eine Okklusionsschiene zur Anbringung an Ober- oder Unterkiefer eines Patienten umfasst einen eine Kaufläche wenigstens eines Zahns (7) des Kiefers überdeckenden und an die Topographie der Kaufläche des Zahns (7) angepassten Distanzkörper (3). In dem Distanzkörper (3) ist wenigstens ein Kanal (19; 25) ausgespart, der einen von dem Distanzkörper (3) überdeckten Punkt (20) der Kaufläche mit der Umgebung verbindet.

## Beschreibung

Okklusionsschienen werden in Zahnheilkunde und Kieferorthopädie zur Korrektur von Fehlstellungen der Zähne und des Kiefergelenks, zur Verhinderung bzw. Therapie von unbewusstem nächtlichem Zähneknirschen, Schnarchen und dergleichen eingesetzt. Sie umfassen im Allgemeinen einen Distanzkörper aus Kunststoff, der wenigstens einen Zahn, meist mehrere Zähne beiderseits einer Mittellinie des Gebisses eines Patienten, überdeckt und dessen Form an einer den Kauflächen der überdeckten Zähne zugewandten Innenfläche so exakt an die Form der Zähne angepasst ist, dass die Okklusionsschiene an den überdeckten Zähnen formschlüssig am Zahnäquator verrastbar ist.

Die formschlussige Verrastung erfordert eine enge Anpassung der Gestalt des Distanzkorpers an die von ihm überdeckten Zähne, mit der Folge, dass, wenn die Okklusionsschiene getragen wird, aufgrund der in einem Spalt zwischen ihr und den überdeckten Zähnen wirksamen Kapillarkrafte ständig ein Speichelfilm in dem Spalt vorhanden ist, dass aber praktisch kein Austausch zwischen diesem Spalt und dem umgebenden Mundraum stattfindet. Wenn die Okklusionsschiene längere Zeit, zum Beispiel über Nacht, kontinuierlich getragen wird, und sich noch organische Reste zwischen den Zähnen und der Schiene befinden, können unter dem Schutz der Schiene Abbauprozesse in Gang kommen, die beim Benutzer einen üblen Geschmack im Mund hervorrufen oder beim Abnehmen der Schiene als unangenehmer Geruch wahrgenommen werden. Hierfür genügen minimale Reste, die auch durch akribische Mundhygiene nur unbefriedigend zu vermeiden sind.

Aufgabe der Erfindung ist, den Tragekomfort einer Okklusionschiene zu verbessern, insbesondere indem die Gefahr eines unangenehmen Geruchs oder Geschmacks wenigstens vermindert ist.

Die Aufgabe wird gelöst, indem bei einer Okklusionsschiene zur Anbringung an Ober- oder Unterkiefer, üblicherweise am Unterkiefer, eines Patienten mit einem eine Kaufläche wenigstens eines Zahns des betreffenden Kiefers überdeckenden und an die Topografie der Kaufläche des Zahns angepassten Distanzkörper, in dem Distanzkörper wengistens ein Kanal ausgespart ist, der einen von dem Distanzkörper überdeckten Punkt der Kaufläche mit der Umgebung verbindet. Ein solcher Kanal ermöglicht, ohne den festen Sitz der Okklusionsschiene am Kiefer zu beeinträchtigen, einen kontinuierlichen Flüssigkeitsaustausch zwischen dem überdeckten Punkt der Kaufläche und dem die Schiene umgebenden Mundraum. Die Oberfläche des überdeckten Zahns ist daher nicht völlig von der Sauerstoffzufuhr aus dem umgebenden Mundraum abgeschlossen, so dass anaerobe Abbauprozesse vermieden werden. Außerdem gewährleistet ein über den Kanal möglicher kontinuierlicher Flüssigkeitsaustausch mit dem umgebenden Mundraum, dass sich Abbauprodukte bzw. die die Abbauprodukte erzeugenden Mikroorganismen nicht zwischen Zahn und Okklusionsschiene sammeln und durch ihre hohe Konzentration wahrnehmbar werden.

Einer ersten Ausgestaltung der Erfindung zu Folge kann der Kanal eine Nut in einer der Kauflache zugewandten Innenfläche des Distanzkörpers sein.

Einer zweiten Ausgestaltung zu Folge ist der Kanal eine Bohrung, die sich von einer der Kaufläche zugewandten Innenfläche des Distanzkörpers durch diesen hindurch zu einer von der Kaufläche abgewandten Außenfläche erstreckt.

Die Anpassung an die Topografie der Kaufläche des Zahns wird im Allgemeinen zumindest darin bestehen, dass die Innenfläche wenigstens eine zu einem Höcker des überdeckten Zahns komplementäre Konkavität aufweist. Um einen effektiven Stoffaustausch entlang des Kanals zu gewährleisten, sollte dieser vorzugsweise von einem Scheitelpunkt der Konkavität ausgehen.

Elastische Verformbarkeit des Distanzkörpers ermöglicht es diesem, einem sich von einander gegenüberliegenden Zähnen ausgeübten Druck nachzugeben und dadurch die Flüssigkeitszirkulation im Kanal anzutreiben.

Um den Flüssigkeitsdurchsatz durch den Kanal zu effektivieren, kann dieser an einem an die Kaufläche angrenzenden Ende eine lokale Aufweitung aufweisen. Je großer deren Querschnitt der Aufweitung ist, um so größer ist auch die Flüssigkeitsmenge, die aus der Aufweitung verdrängt wird, wenn die Okklusionsschiene Druck von einander gegenüberliegenden Zähnen ausgesetzt ist, und um so stärker ist folglich die Zirkulation in dem Kanal.

Der Distanzkörper ist vorzugsweise an seiner von der Kaufläche abgewandten Außenfläche wenigstens lokal flach. Dies hat zur Folge, dass die mechanische Belastung und auch eine aus dieser Belastung resultierende Verformung des Distanzkörpers dort am größten ist, wo ein Höcker eines gegenüberliegenden Zahns die Außenfläche berührt. Wenn der Kanal entfernt von diesen maximal belasteten Bereichen, an der Innenseite des Distanzkörpers oder ausgehend von einem Scheitelpunkt Konkavität quer durch den Distanzkörper hindurch verläuft, ist die Gefahr gering, dass durch die Verformung des Distanzkörpers auch der Kanal verengt wird.

Typischerweise umfasst die Okklusionsschiene einen zweiten Distanzkörper und ein die beiden Distanzkörper verbindendes bogenförmiges Mittelstück, wobei einer der Distanzkörper zur Anbringung an wenigstens einem rechten Backenzahn des Kiefers und der andere Distanzkörper zur Anbringung an wenigstens einem linken Backenzahn eines Kiefers ausgebildet ist und das Mittelstück Schneidezähne des Kiefers lingual berührt.

Die Inzisalkante der Schneidezähne sollte vom Mittelstück unbedeckt bleiben, um die Beweglichkeit der Zunge und damit die Artikulationsfähigkeit eines Benutzers der Schiene nicht einzuschränken.

Mittelstück und Distanzkörper sind typischerweise einteilig aus Kunstharz geformt.

Der Distanzkörper kann an seinem vorderen Ende eine Erhebung tragen, die im Zusammenwirken mit Zähnen des gegenüberliegenden Kiefers eine Bewegung des Unterkiefers führen bzw. eine Ruhestellung des Unterkiefers festlegen kann.

Wenn die Okklusionsschiene zur Anbringung am Unterkiefer ausgebildet ist, kann die Erhebung insbesondere eine erste Flanke aufweisen, die in Schließstellung des Unterkiefers die palatinale Flanke eines Eckzahns des Oberkiefers berührt, und eine zweite Flanke aufweisen, die in der Schließstellung die mesiale Flanke eines ersten Prämolars des Oberkiefers berührt.

Weitere Merkmale und Vorteile der Erfindung ergeben sich aus der nachfolgenden Beschreibung von Ausführungsbeispielen unter Bezugnahme auf die beigefugten Figuren. Es zeigen:
- Fig. 1: einen Unterkiefer mit einer erfindungsgemaßen Okklusionsschiene;
- Fig. 2: einen Distanzkörper der Okklusionsschiene im Querschnitt gemäß einer ersten Ausgestaltung der Erfindung;
- Fig. 3: den Distanzkörper im Querschnitt gemessenen zweiten Ausgestaltung; und
- Fig. 4: den Distanzkörper im Querschnitt gemäß einer vierten Ausgestaltung.

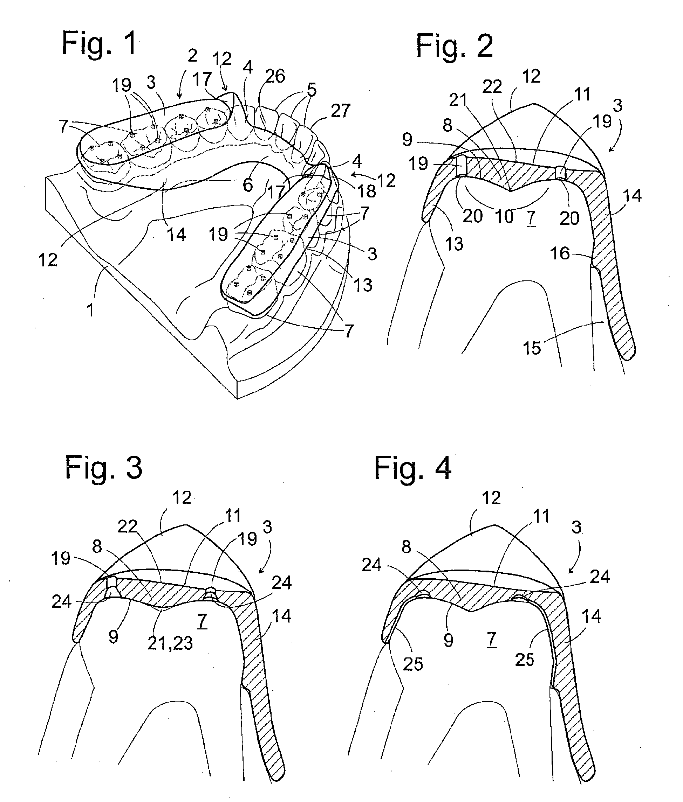

Fig. 1 zeigt in einer perspektivischen Ansicht einen Unterkieferabguss 1 eines Patienten mit einer darauf montierten Okklusionsschiene 2. Die Okklusionsschiene 2 ist im einfachsten Fall ein einteiliger Körper aus homogenem Kunststoff mit zwei seitlichen Distanzkorpern 3 und einem die Distanzkörper 3 verbindenden, sich entlang der lingualen Flanken von Eckzähnen 4 und Schneidezähnen 5 bogenförmig erstreckenden Mittelstück 6. Die Distanzkör - per 3 überdecken alle vorhandenen Backenzähne 7, hier je vier Stuck auf der linken und der rechten Seite, oder zumindest diejenigen Backenzähne 7, die ein Gegenstück im Oberkiefer haben.

Fig. 2 zeigt einen Distanzkörper 3 im Querschnitt. Eine im Wesentlichen horizontale zentrale Platte 8 des Distanzkörpers 3 liegt auf den Kauflächen der Backenzähne 7 auf und ist an ihrer den Backenzähnen zugewandten Innenfläche 9 mit die Höcker der Backenzähne 7 formschlüssig aufnehmenden Einbuchtungen 10 versehen. Die Oberseite der Platte 8 ist zum großen Teil durch eine im Wesentlichen ebene Außenfläche 11 gebildet; zur Kauflächenkontur der Backenzähne des Oberkiefers komplementäre Einbuchtungen sind nicht vorhanden. Lediglich an den mesialen Enden der Distanzkörper 3 ist jeweils eine pyramiden- oder tetraederähnliche Erhebung 12 geformt, die über die ebene Außenfläche 11 aufragt.

Seitliche Schenkel 13, 14 der Distanzkörper 3 liegen an den Backzähnen 7 bukkal bzw. lingual an, um einen festen Halt der Schiene 2 auch bei geöffnetem Mund zu gewährleisten. Der linguale Schenkel 14 erstreckt sich bis auf das apikal an den Backenzahn 7 angrenzende Zahnfleisch 15. In Hohe des Zahnhalses kann der Schenkel 14 mit einer flachen, am Zahn 7 apikal von dessen Zahnäquator und am angrenzenden Zahnfleisch 15 anliegenden Rippe 16 versehen sein, die zusammen mit einer spiegelbildlichen Rippe 16 am gegenüberliegenden Distanzkörper 3 eine formschlüssige Verrastung der Schiene 2 an den Zähnen des Unterkiefers ermöglicht.

Wie mit Bezug auf Fig. 1 deutlich wird, enden die Distanzkörper 3 mesial in Höhe der Eckzähne 4, und das Mittelstück 6 erstreckt sich im Wesentlichen in Verlängerung der lingualen Schenkel 14 der Distanzkörper 3.

Das Mittelstück 6 hat einen stegformig langgestreckten, beiderseits des Zahnhalses des Schneidezahns 5 an dessen lingualer Flanke 26 sowie am angrenzenden Zahnfleisch 15 anliegenden Querschnitt. Der von dem Mittelstück 6 überdeckte Bereich der lingualen Flanke 26 nimmt hier knapp über die Hälfte von deren Hohe ein; der Rest der Flanke 26, bis hin zur Inzisalkante 27, liegt frei und kann von der Zunge beruhrt werden. Da die Flanke 26 gegen die Richtung der Kieferbewegung geneigt ist, ist der Schneidezahn 5 in dem Bereich 16 jedes Mal Druck durch das daran anliegende Mittelstück 6 ausgesetzt, wenn Zähne des Oberkiefers auf die Distanzkörper 3 drücken. So bekommt der Zahn 5 regelmäßig den Druckreiz, der erforderlich ist, um eine Verschiebung des Zahns 5 im Kiefer zu vermeiden.

Die benachbart zu den Eckzähnen 4 aufragenden Erhebungen 12 sind vorgesehen, um in der Schließstellung des Unterkiefers, d.h. wenn Zähne des Ober- und Unterkiefers an den Distanzkörpern 3 anliegen, zwischen die palatinale Flanke von Eckzähnen des Oberkiefers und die mesiale Flanke der ersten Pramolaren des Oberkiefers einzugreifen und dabei beide zu berühren. Die Erhebungen 9 haben jeweils eine distale schräge Flanke 17, die vorgesehen ist, um in der Schließstellung den ersten Prämolar zu berühren, und eine bukkale Flanke 18, die in der Schließstellung gegen den Eckzahn stößt.

Würde man eine zusätzliche Materialschicht auf die distale Flanke 17 der beiden Distanzkörper 3 aufbringen oder die Erhebungen 12 nach distal verschieben, so hatte dies zur Folge, dass beim Zubeißen der Kontakt der Flanken 17 mit den ersten Prämolaren früher zustande kommt, und bevor die übrigen Backenzähne an die Außenfläche 11 der Distanzkörper anschlagen, wird durch den Kontakt mit der Flanke 17 der Unterkiefer nach vorn verschoben. Entsprechend ist durch Materialauftrag auf den bukkalen Flanken 18 oder Verschiebung der Erhebungen 12 nach mesial ein früherer Kontakt mit den Eckzahnen des Oberkiefers erreichbar, durch den der Unterkiefer bis zum Erreichen der Schließstellung rückwarts verschoben wird. In entsprechender Weise ist durch unterschiedlichen Materlalaüf- oder - abtrag an den bukkalen Flanken 18 beider Distanzkörper 3 oder durch Seitwärtsverschiebung beider Erhebungen 12 in derselben Richtung eine Drehung der Achse des Kiefergelenks erreichbar. So können durch eine passende Formgebung der Erhebungen 12 diverse Kieferfehlsteilungen korrigiert und Gelenküberlastungen vermieden werden.

Zwischen den Einbuchtungen 10 der Innenfläche 9 und der Außenfläche 11 erstrecken sich zahlreiche Bohrungen 19 im Wesentlichen vertikal durch die Distanzkörper 3.

Die Bohrungen 19 haben einen Durchmesser von unter einem Millimeter, vorzugsweise von 0,2 bis 0.5 mm. Da sie nicht wie die Innenfläche 9 vom Kiefer eines Patienten abgeformt werden können, werden sie nachträglich, durch mechanisches Bohren oder durch Laserablation, in die Distanzkörper 3 eingebracht.

Die hier im Schnitt gezeigten Bohrungen 19 erstrecken sich durch den Distanzkörper 3 jeweils an dessen dunnsten Stellen, zwischen der Außenfläche 11 und Scheitelpunkten 20 der Einbuchtungen 10. Da die Einbuchtungen 10 jeweils von einem Höcker eines vom Distanzkörper 3 überdeckten Zahns 7 abgeformt sind und die Molaren vier, die Prämolaren zwei Höcker haben, entfallen je vier Bohrungen 19 auf einen überdeckten Molar und zwei Bohrungen 19 auf einen überdeckten Prämolar.

Der in Fig. 2 im Schnitt gezeigte Backenzahn 7 des Unterkiefers hat zwischen den zwei gezeigten Höckern eine Vertiefung 21, in die, wenn die Okklusionsschiene 2 nicht vorhanden wäre, in einer Schließstellung der Kiefer ein Höcker eines gegenüberliegenden Backenzahns des Oberkiefers eingreifen wurde. Wenn der Backenzahn 7 des Unterkiefers wie in der Figur gezeigt von der Okklusionsschiene 2 uberdeckt ist, trifft der Höcker des gegenüberliegenden Oberkiefer-Backenzahns beim Schließen der Kiefer oberhalb der Vertiefung 15 in einem Bereich 22 der Außenflache 11 auf die Außenfläche 11 des Distanzkörpers 3. Der dadurch auf den Distanzkörper 3 ausgeübte Druck zwingt den Distanzkörper 3 insbesondere im Bereich der Vertiefung 15 in unmittelbaren Kontakt mit dem Backenzahn 7, so dass in diesem Bereich zwischen Distanzkörper 3 und Backenzahn 7 eventuell noch vorhandener Speichel verdrängt wird und in einen weniger beanspruchten Bereich, insbesondere zu den Scheitelpunkten 20, ausweicht und von dort über die Bohrungen 19 abfließen kann. Wenn der Distanzkörper 3 entlastet wird und sich entspannt, fließt der Speichel durch die Bohrungen 19 zurück, so dass bei jedem Zusammenbeißen der Kiefer der Spalt zwischen Distanzkörper 3 und Zahn 7 gespült wird.

In der Ausgestaltung der Fig. 3 ist dieser Spülungseffekt durch zwei unabhängig voneinander realisierbare Merkmale noch verstärkt. Dem ersten Merkmal zu Folge ist die Vertiefung 21 des Zahns 7 nicht vollständig von dem Distanzkörper 3 ausgefüllt, sondern der Spalt zwischen Zahn 7 und Distanzkörper 3 ist im Bereich der Vertiefung 15 zu einem kleinen Zwischenraum 23 aufgeweitet, der, wenn der Hocker des gegenüberliegenden Zahns auf die Außenfläche 11 druckt, unter Druck gerät, so dass Flüssigkeit aus diesem Zwischenraum 23 zu den Bohrungen 19 verdrängt wird

Dem zweiten Merkmal zu Folge sind die Bohrungen 19 an ihrer der Innenfläche 9 zugewandten Seite mit Aufweitungen 24 versehen, um eine Stauchung der Distanzkörper 3 unter Druck und damit die Verdrängung von Flüssigkeit von den Höckern des Zahns 7 zu intensivieren. Da der Durchmesser der Bohrungen 19 an der Außenfläche- 11 unverändert klein bleibt, wird gleichzeitig die Wahrscheinlichkeit gering gehalten, dass Partikel aus abbaubarem organischem Material durch die Bohrungen 19 zum Zahn 7 gelangen, wenn der Distanzkörper 3 sich entspannt und dabei Speichel durch die Bohrungen 19 in den Spalt zwischen Innenfläche 8 und Zahn 7 einströmt.

Nicht völlig auszuschließen ist die Möglichkeit, dass organisches Material unter dem Druck von Zähnen des gegenüberliegenden Kiefers in die Bohrungen 19 eingepresst wird und so in den Spalt zwischen Innenfläche 8 und Zahn 7 gelangt oder in einer Bohrung 11 stecken bleibt. Diese Möglichkeit ist weitgehend vermieden durch eine in Fig. 4 gezeigte weitere Variante des Distanzkörpers 3, bei der die Bohrungen 19 ersetzt sind durch Nuten 25, die sich an der Innenfläche 9 des Distanzkorpers 3 jeweils von einem Scheitelpunkt 20 zu einem benachbarten Rand der Innenfläche 9 erstrecken. Auch die Nuten 25' sind an der durch Abformen vom Gebiss des Patienten erhaltenen Innenfläche 9 nachträglich, insbesondere durch Fräsen oder Laserablation, einigebracht. Indem sie entfernt von den Kauflächen der Zähne 7, zwischen einem Rand des Distanzkörpers 3 und dem Zahnfleisch, in die Mundhöhle münden, sind sie gut vor dem Eindringen von organischem Material geschützt; außerdem ist solches Material, wenn es in die Nuten 25 gelangt sein sollte, leichter wieder zu beseitigen als aus einer Bohrung.

Auch hier können die Enden der Nuten 25 an den Scheitelpunkten 12 der Konkavitaten 13 jeweils mit Aufweitungen 24 versehen sein, um die Menge der Flüssigkeit, die bei Ausüben von Druck auf den Distanzkörper 3 zwischen diesem und dem Zahn 7 entweicht bzw. nach Druckentlastung in den Zwischenraum zurückkehrt, zu vergrößern.

### Bezugszeichen

- 1: Abguss
- 2: Okklusionsschiene
- 3: Distanzkörper
- 4: Eckzahn
- 5: Schneidezahn
- 6: Mittelstück
- 7: Backenzahn
- 8: Platte
- 9: Innenfläche
- 10: Einbuchtung
- 11: Außenfläche
- 12: Erhebung
- 13: bukkaler Schenkel
- 14: lingualer Schenkel
- 15: Zahnfleisch
- 16: Rippe
- 17: Flanke
- 18: Flanke
- 19: Bohrung
- 20: Scheitelpunkt
- 21: Vertiefung
- 22: Bereich
- 23: Zwischenraum
- 24: Aufweitung
- 25: Nut
- 26: Flanke
- 27: Inzisalkante

## Patentansprüche

1. Okklusionsschiene zur Anbringung an Ober- oder Unterkiefer eines Patienten, mit einem eine Kaufläche wenigstens eines Zahns (7) des Kiefers überdeckenden und an die Topographie der Kaufläche des Zahns (7) angepassten Distanzkörper (3), **dadurch gekennzeichnet, dass** in dem Distanzkörper (3) wenigstens ein Kanal (19; 25) ausgespart, ist, der einen von dem Distanzkörper (3) überdeckten Punkt der Kaufläche mit der Umgebung verbindet.

2. Okklusionsschiene nach Anspruch 1, **dadurch gekennzeichnet, dass** der Kanal eine Nut (25) in einer der Kaufläche zugewandten Innenfläche (9) des Distanzkörpers (3) ist.

3. Okklusionsschiene nach Anspruch 1, **dadurch gekennzeichnet, dass** der Kanal eine Bohrung (19) ist, die sich von einer der Kaufläche zugewandten Innenfläche (9) des Distanzkörpers (3) zu einer von der Kaufläche abgewandten Außenfläche (11) des Distanzkörpers (3) erstreckt.

4. Okklusionsschiene nach einem der vorhergehenden Ansprüche, **dadurch gekennzeichnet, dass** die Innenfläche (9) wenigstens eine zu einem Höcker des überdeckten Zahns komplementäre Einbuchtung (10) aufweist und der Kanal (19; 25) von einem Scheitelpunkt (20) der Einbuchtung (10) ausgeht.

5. Okklusionsschiene nach einem der vorhergehenden Ansprüche, **dadurch gekennzeichnet, dass** der Distanzkörper (3) elastisch verformbar ist.

6. Okklusionsschiene nach einem der vorhergehenden Ansprüche, **dadurch gekennzeichnet, dass** der Kanal (19; 25) an einem an die Kaufläche angrenzenden Ende eine lokale Aufweitung (24) aufweist.

7. Okklusionsschiene nach einem der vorhergehenden Ansprüche, **dadurch gekennzeichnet, dass** der Distanzkörper (3) an seiner von der Kaufläche abgewandten Außenfläche (11) wenigstens lokal flach ist.

8. Okklusionsschiene nach einem der vorhergehenden Ansprüche, **dadurch gekennzeichnet, dass** sie einen zweiten Distanzkörper (3) und ein die Distanzkörper (3) verbindendes bogenförmiges Mittelstück (6) umfasst, wobei einer der Distanzkörper (3) zur Anbringung an wenigstens einem rechten Backenzahn (7) des Kiefers und der andere Distanzkörper (3) zur Anbringung an wenigstens einem linken Backenzahn (7) des Kiefers ausgebildet ist und das Mittelstück (6) Schneidezähne (5) des Kiefers lingual berührt.

9. Okklusionsschiene nach Anspruch 8, **dadurch gekennzeichnet, dass** das Mittelstück (6) die Inzisalkante (27) der Schneidezähne (5) unbedeckt lässt.

10. Okklusionsschiene nach Anspruch 8 oder 9, **dadurch gekennzeichnet, dass** das Mittelstück (6) und die Distanzkörper (3) einteilig aus Kunstharz geformt sind.

11. Okklusionsschiene nach einem der vorhergehenden Ansprüche, **dadurch gekennzeichnet, dass** der Distanzkörper (3) an seinem vorderen Ende eine Erhebung (12) trägt.

12. Okklusionsschiene nach Anspruch 11, **dadurch gekennzeichnet, dass** sie zur Anbringung am Unterkiefer ausgebildet ist und die Erhebung (12) eine erste Flanke (18) aufweist, die in Schließstellung des Unterkiefers die palatale Flanke eines Eckzahns des Oberkiefers berührt, und eine zweite Flanke (17) aufweist, die in der Schließstellung die mesiale Flanke eines ersten Prämolars des Oberkiefers berührt.
